# EUROPEAN PATENT SPECIFICATION

(11) **EP 1 938 908 B1**
(45) Date of publication and mention of the grant of the patent: **12.05.2010**
(21) Application number: 06127352.0
(22) Date of filing: 29.12.2006
(51) Int. Cl.: B06B 3/00, A61N 7/00

(54) **Method for manufacturing a membrane and object provided with such a membrane**
Herstellungsverfahren für eine Membran und mit einer solchen Membran versehener Gegenstand
Procédé de fabrication d'une membrane et objet doté d'une telle membrane

(43) Date of publication of application: 02.07.2008
(73) Proprietor: Ultrazonix DNT AB, 216 16 Limhamn (SE)
(72) Inventor: Olsson, Thord, 227 32, Lund (SE); Lidgren, Lars, 222 21, Lund (SE); Forslund, Carina, 241 31 Eslöv (SE)
(74) Representative: Schwarze, Holm

(56) References cited:
- EP-A- 1 769 854
- WO-A-97/23865
- DE-A1- 19 742 294
- US-A- 5 550 790

## Description

### Field of the invention

The present invention relates to a method for manufacturing a membrane or wall of a material, which is capable of vapour/vacuum deposition, over an opening of an object. The invention also relates to such an object provided with a membrane, and particularly an ultrasonic transducer provided with a front membrane.

### Background of the invention

In medical applications, it is known to coat devices to be introduced in the human body to protect the body from e.g. metallic contact. For example pacemakers are usually coated with Parylene, which is a depositable material well tolerable to the human body.

Ultrasonic probes for insertion in the human body for various treatments are also known. They may require cooling of the ultrasonic transducer. Also, they may require a liquid in front of the ultrasonic probe to assist in coupling the ultrasonic radiation to tissue to be treated. For this reason it has been contemplated to provide a chamber at the front end of the ultrasonic probe to distribute cooling liquid, typically saline, in front of an ultrasonic transducer.

The problem is to form a wall over the opening of the probe in an easy and efficient way.

Document DE 19742294 describes an ultrasound arrangement and a method to manufacture it.

### Summary of the invention

One purpose of the invention is to provide a method for manufacturing a membrane over an opening in a simple and efficient way, the membrane having the required strength and bonding to the probe body.

A further purpose is to provide a method for manufacturing a membrane of a material tolerable to the human body on an ultrasonic transducer.

A further purpose is to provide an object provided with such a membrane.

In a first aspect, the invention provides a method for manufacturing a membrane of a depositable material at an opening of an object.

The method includes:
placing the opening with a defined rim against a backing surface coated with a layer repellent to the depositable material;
depositing of the depositable material on the object and the backing surface to form an element;
separation of the element from the backing surface leaving a membrane integral with the element.

Suitably, at least one hole is manufactured in the element by arranging a recess in the rim, placing the recess against the backing surface, so that the depositable material is deposited on surfaces of the recess and the backing surface, leaving a hole in the element adjacent the membrane after the separation.

The depositable material may be Parylene , and the repellent layer may be a wax coating.

In a second aspect, the invention provides an object as defined in claim 7. The object comprises a membrane of a depositable material at an opening, the membrane being attached to a defined rim of the opening and being integral with an element deposited on the object.

In one embodiment, the object is an ultrasonic transducer.

The membrane may form a front wall of a chamber arranged between an ultrasonic crystal and said wall.

The invention is defined in claims 1 and 7, while embodiments are set forth in the dependent claims.

### Brief description of the drawings

The invention will be described in detail below with reference to the accompanying drawings, of which:
Fig. 1 is a perspective view of an ultrasonic transducer before manufacturing the membrane,
Fig. 2 is a front view of the transducer of fig. 1,
Fig. 3 is a cross sectional view of the transducer and the backing surface in a step of the manufacturing process, and
Fig. 4 is a perspective view of an ultrasonic transducer provided with a membrane according to an embodiment of the invention.

### Detailed description of preferred embodiments

The invention relates generally to a method for manufacturing a membrane applicable to objects with one or more openings to be covered with a membrane using a depositable material. The invention will be described with reference to a specific example of arranging a membrane on an ultrasonic probe or transducer, but it will be appreciated that the method is equally applicable to other objects.

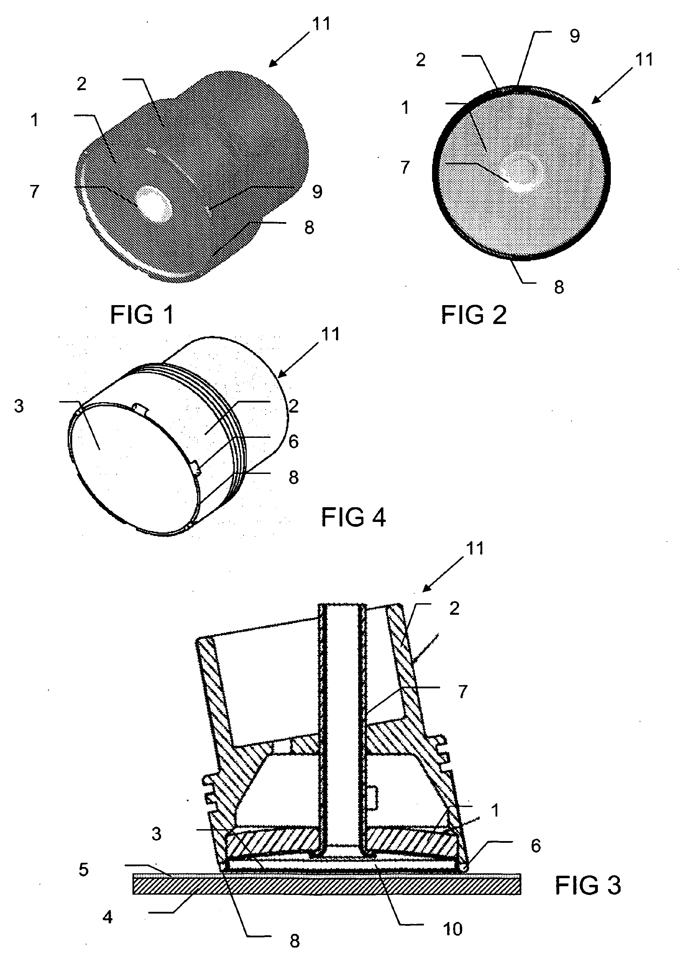

The method was developed for arranging a membrane on an ultrasonic probe. An ultrasonic probe comprises a handle, a generally tubular introducer, electric wires, fluid lines etcetera (not shown) connected to a transducer. A schematic, exemplary ultrasonic transducer 11 is shown in the drawings. The transducer 11 comprises a probe head 2 carrying an ultrasonic crystal 1 and a conduit 7 for conducting cooling fluid to a region generally in front of the crystal 1. Cooling fluid should be distributed over the front surface, to the right in fig. 3, of the crystal 1. Thus, a front wall or membrane 3 is needed.

In the present specification, the term depositable material is intended to mean any material that is capable of vapour/vacuum deposition. Parylene is a suitable choice for ultrasonic probes.

Parylene is the generic name for the poly-para-xylylenes. Parylene is a conformal protective polymer coating material utilized to uniformly protect any component configuration on such diverse substrates as metal, glass, paper, resin, plastic, ceramic, ferrite and silicon. Because of its unique properties, Parylene conforms to virtually any shape, including sharp edges, crevices, points; or flat and exposed internal surfaces.

In an embodiment of the invention, the method comprises the following steps. The transducer 11 is placed on a backing surface 4 provided with a repellent layer, in case of Parylene as depositable material, preferably a wax coating. The backing surface is suitably horizontal so that the transducer 11 is held in place by gravity. Alternatively, the backing surface is placed on top of the transducer 11 or they are clamped together by means of other arrangements. Several transducers 11 may be provided with membranes simultaneously. The front opening of the probe head 2 has a rim 8 in contact with the repellent layer 5.

The backing surface 4 with the transducer 11 is placed in a vacuum chamber. Evaporation of the Parylene may be performed according to known techniques. The evaporated Parylene enters between the crystal 1 and the backing surface through the conduit 7. The Parylene will cover all surfaces of the transducer 11 and the repellent layer 5 on the backing surface 4. The evaporation continuous until the membrane 3 formed on the backing surface reaches a sufficient thickness. The thickness should be sufficiently great to provide the necessary strength, but in case of an ultrasonic probe, a too great thickness could result in unwanted attenuation losses of the transmitted ultrasound. The penetration depth of the ultrasound depends on the ultrasound frequency. A thickness in the range of 20 to 30 micrometers, for example around 25 micrometers has been found adequate with an ultrasound frequency around 4 MHz.

In the figures only the membrane 3 is shown, but the Parylene forms a continuous integral element or body covering also the other surfaces of the transducer 11 including the front surface of the crystal 1. Thus, a chamber 10 is formed by the Parylene material in front of the crystal 1.

Thanks to the evaporation process, there are no seams and sharp corners in the Parylene material and all transitions between portions of the Parylene body are smooth. Thus, the membrane is bonded with high strength to the Parylene body attached to the probe head 2, the crystal 1 and other surfaces of the transducer 11.

To separate the transducer 11 from the backing surface 4, a cut is performed around the probe head 2. Then the transducer 11 may be pulled from the backing surface, as the membrane is only weakly adhered to the repellent layer 5. Suitably, the edges round the rim 8 are trimmed to a desired smooth shape.

In case of cooling of an ultrasonic probe, the chamber 10 needs at least one opening 6 to discharge the cooling fluid. To produce openings in the Parylene material, at least one recess 9 is provided in the rim 8. Suitably, a number of recesses 9 are distributed over the rim 8. When the rim 8 is placed against the backing surface, the recess 9 will form an opening. In the evaporation process the depositable material will coat the surfaces of the recess and the backing surface thus forming a continuous material layer of Parylene forming the opening 6. Thus, the hole 6 is defined by the coated surfaces of the recess 9 and by the membrane 3. There are no seams and sharp corners in the hole 6, resulting in a hole resistant to any tearing forces.

In the embodiment shown the backing surface 4 is flat. It will be appreciated that concave and convex shapes may be used as well for forming other desired shapes of the membrane in dependence of the intended application.

Also, the method is applicable to manufacturing a membrane on a general object comprising an opening. Generally the whole object is covered by the depositable material as well, and trimmings may be performed as required. In case of an ultrasonic probe, it is in any case desired that the whole transducer is covered by Parylene, even if the probe head usually is made of plastic.

The method may be used with any material capable of deposition and forming a continuous membrane, e.g. plastics and metal. The repellent surface is selected in dependence of the material to be deposited. For example, wax and Teflon^{™} may be used. The scope of the invention is only limited by the claims below.

## Claims

1. A method for manufacturing a membrane (3) of a depositable material at an opening of an object (2), **characterised by**
placing the opening with a defined rim (8) against a backing surface (4) coated with a layer (5) repellent to the depositable material;
depositing the depositable material on the object (2) and the backing surface (4) to form an element; and
separating the element from the backing surface (4) leaving a membrane (3) integral with the element.

2. A method according to claim 1, wherein the element is separated from the backing surface (4) by cutting the deposited material around the opening, and pulling the object (2) with the element from the backing surface (4).

3. A method according to claim 1 or 2, wherein at least one hole (6) is manufactured in the element by arranging a recess (9) in the rim (8), placing the recess (9) against the backing surface (4), so that the depositable material is deposited on surfaces of the recess (9) and the backing surface (4), leaving a hole (6) in the element adjacent the membrane (3) after the separation.

4. A method according to claim 1, 2 or 3, wherein the depositable material is deposited by means of vacuum deposition.

5. A method according to claim 4, wherein the depositable material is Parylene.

6. A method according to claim 4 or 5, wherein the repellent layer (5) comprises a wax coating.

7. An object comprising at least one opening **characterised in that** the object comprises a membrane (3) of a depositable material arranged to cover said at least one opening, the membrane (3) being attached to a defined rim (8) of the opening and said membrane being integral at said defined rim (8) with a continuous element of the depositable material deposited on and covering the object (2) so as to form a chamber (10) by the deposited material.

8. An object according to claim 7, comprising at least one hole (6) manufactured in the element adjacent the membrane (3), wherein the edges of the hole (6) are defined by depositable material deposited on surfaces of at least one recess (9) provided in the rim (8) and by the membrane (3).

9. An object according to claim 7 or 8, wherein the depositable material is Parylene.

10. An object according to claim 8 or 9, wherein the object is an ultrasonic transducer (11).

11. An object according to claim 10, wherein the membrane (3) forms a front wall of a chamber (10) arranged between an ultrasonic crystal (1) and said wall (3).

12. An object according to claim 10, wherein the membrane (3) has a thickness in the range of 20 to 30 micrometers.

13. An object according to claim 11 or 12, further comprising a conduit for conducting cooling fluid into said chamber.

## Patentansprüche

1. Verfahren zur Herstellung einer Membran (3) eines ablagerbaren Materials an einer Öffnung eines Gegenstands (2), **dadurch gekennzeichnet, dass**
die Öffnung mit einem definierten Rand (8) an eine Unterstützungsfläche (4) angelegt wird, welche mit einer das ablagerbare Material abweisenden Schicht (5) überzogen ist;
das ablagerbare Material zur Bildung eines Elements an der Öffnung (2) und der Unterstützungsfläche (4) abgelagert wird; und
das Element eine mit dem Element integrierte Membran (3) hinterlassend von der Unterstützungsfläche (4) getrennt wird.

2. Verfahren nach Anspruch 1, wobei das Element von der Unterstützungsfläche (4) durch Schneiden des abgelagerten Materials um die Öffnung herum und Wegziehen des Gegenstands (2) mit dem Element von der Unterstützungsfläche (4) getrennt wird.

3. Verfahren nach Anspruch 1 oder 2, wobei zumindest ein Loch (6) durch Anbringen einer Aussparung (9) im Rand (8) im Element hergestellt wird, indem die Aussparung (9) an die Unterstützungsfläche (4) angelegt wird, so dass das ablagerbare Material auf Oberflächen der Aussparung (9) und der Unterstützungsfläche (4) neben der Membran (3) nach dem Trennen ein Loch (6) in dem Element hinterlassend abgelagert wird.

4. Verfahren nach Anspruch 1, 2 oder 3, wobei das ablagerbare Material mittels Vakuumablagerung abgelagert wird.

5. Verfahren nach Anspruch 4, wobei das ablagerbare Material Parylen ist.

6. Verfahren nach Anspruch 4 oder 5, wobei die abweisende Schicht (5) eine Wachsbeschichtung umfasst.

7. Gegenstand, umfassend zumindest eine Öffnung,
**dadurch gekennzeichnet, dass** der Gegenstand eine Membran (3) eines ablagerbaren Materials umfasst, welches zur Abdeckung der zumindest einen Öffnung eingerichtet ist, welche Membran (3) an einem definierten Rand (8) der Öffnung befestigt ist, und wobei die Membran zur Bildung einer Kammer (10) durch das abgelagerte Material am definierten Rand (8) mit einem kontinuierlichen Element des am Gegenstand (2) abgelagerten und denselben abdeckenden ablagerbaren Materials integriert ist.

8. Gegenstand nach Anspruch 7, welcher Gegenstand zumindest ein neben der Membran (3) in dem Element hergestelltes Loch (6) umfasst, wobei die Kanten des Lochs (6) durch das ablagerbare Material, welches auf Oberflächen zumindest einer im Rand (8) vorgesehenen Aussparung (9) abgelagert wird, und durch die Membran (3) definiert sind.

9. Gegenstand nach Anspruch 7 oder 8, wobei das ablagerbare Material Parylen ist.

10. Gegenstand nach Anspruch 8 oder 9, wobei der Gegenstand ein Ultraschallwandler (11) ist.

11. Gegenstand nach Anspruch 10, wobei die Membran (3) eine Vorderwand einer zwischen einem Ultraschallkristall (1) und der Wand (3) angebrachten Kammer (10) bildet.

12. Gegenstand nach Anspruch 10, wobei die Membran (3) eine Dicke im Bereich von 20 bis 30 Mikrometern aufweist.

13. Gegenstand nach Anspruch 11 oder 12, welcher Gegenstand eine Leitung zur Führung eines Kühlfluids in die Kammer umfasst.

## Revendications

1. Procédé de fabrication d'une membrane (3) en un matériau apte à être déposé sur l'ouverture d'un objet (2), **caractérisé par** les étapes qui consistent à
placer l'ouverture dotée d'une bordure (8) définie contre une surface de dos (4) revêtue d'une couche (5) répulsive vis-à-vis du matériau apte à être déposé,
déposer le matériau apte à être déposé sur l'objet (2) et sur la surface de dos (4) pour former un élément et
séparer l'élément de la surface de dos (4) en conservant une membrane (3) formée d'un seul tenant avec l'élément.

2. Procédé selon la revendication 1, dans lequel l'élément est séparé de la surface de dos (4) en découpant le matériau déposé autour de l'ouverture et en tirant l'objet (2) et la surface de dos (4) à l'aide de l'élément.

3. Procédé selon les revendications 1 ou 2, dans lequel au moins un trou (6) est ménagé dans l'élément en prévoyant un creux (9) dans la bordure (8) et en plaçant le creux (9) contre la surface de dos (4) de telle sorte que le matériau apte à être déposé puisse être déposé sur la surface du creux (9) et la surface de dos (4) en laissant après la séparation et en position adjacente à la membrane (3) un trou (6) dans l'élément.

4. Procédé selon les revendications 1, 2 ou 3, dans lequel le matériau apte à être déposé est déposé par dépôt sous vide.

5. Procédé selon la revendication 4, dans lequel le matériau apte à être déposé est le parylène.

6. Procédé selon les revendications 4 ou 5, dans lequel la couche répulsive (5) comporte un revêtement de cire.

7. Objet comprenant au moins une ouverture, **caractérisé en ce que** l'objet comprend une membrane (3) de matériau apte à être déposé agencée de manière à couvrir la ou lesdites ouvertures, **en ce que** la membrane (3) est fixée à une bordure définie (8) de l'ouverture et **en ce que** sur ladite bordure définie (8), ladite membrane est d'un seul tenant avec un élément continu de matériau apte à être déposé déposé sur l'objet (2) et le recouvre de manière à former une chambre (10) par le matériau déposé.

8. Objet selon la revendication 7, comprenant au moins un trou (6) ménagé dans l'élément en position adjacente à la membrane (3), les bords du trou (6) étant définis par un matériau apte à être déposé déposé sur la surface d'au moins un creux (9) prévu dans la bordure (8) et par la membrane (3).

9. Objet selon les revendications 7 ou 8, dans lequel le matériau apte à être déposé est le parylène.

10. Objet selon les revendications 8 ou 9, dans lequel l'objet est un transducteur (11) à ultrasons.

11. Objet selon la revendication 10, dans lequel la membrane (3) forme la paroi avant d'une chambre (10) prévue entre un cristal ultrasonique (1) et ladite paroi (3).

12. Objet selon la revendication 10, dans lequel la membrane (3) a une épaisseur de l'ordre de 20 à 30 micromètres.

13. Objet selon les revendications 11 ou 12, comprenant en outre un conduit qui conduit un fluide de refroidissement dans ladite chambre.
